# EUROPEAN PATENT APPLICATION

(11) **EP 0 596 840 A1**
(43) Date of publication of application: **11.05.1994**
(21) Application number: 93830426.8
(22) Date of filing: 26.10.1993
(51) Int. Cl.: G01N 33/50, G01N 33/569

(54) **Method for performing a lymphocyte transformation test by flow cytometry**

(30) Priority: 05.11.1992 IT RM920803
(71) Applicant: Bussa, Stefano, I-00152 Roma RM (IT); Rumi, Carlo, I-00195 Roma RM (IT)
(72) Inventor: Bussa, Stefano, I-00152 Roma RM (IT); Rumi, Carlo, I-00195 Roma RM (IT)
(74) Representative: Di Cerbo, Mario

(57) **Abstract**

Method for performing a stimulated lymphocyte transformation test, comprising taking a sterile heparinated sample, implanting the whole blood or separated lymphocytes in culture medium, incubation in a sterile incubator, setting up two cultures, one of which must contain the stimulant at concentrations such as to generate cell proliferation,
characterized by the fact that incubation takes place at a temperature of between 25 and 45°C for a time of between 24 and 192 hours, and by the fact that it comprises the combination of the following operations:
- centrifugation at the end of the incubation;
- re-suspension in a lysing solution for erythrocytes;
- DNA staining;
- washing the leucocyte suspension with isotonic aqueous solution;
- centrifugation of the resulting leucocyte suspension;
- re-suspension of the cells in a solution lysing for leucocytes, in the presence of a DNA-specific fluorochrome, the lysing solution also optionally comprising reagents for optimization of lysis;
- analysis of the samples, using flow cytometer.

## Description

The present invention relates to a method for performing a stimulated lymphocyte transformation test (generally speaking a cell transformation test) by flow cytometry. This method, both because of its simplicity and versatility, is suitable for clinical use, unlike other conventional methods, which because of their complexity and delicate nature are used mainly in research laboratories.

As is known, the activation or stimulation of lymphocytes refers to an in vitro correlate of a process occurring regularly in vivo when the antigen reacts with sensitized lymphocytes. Lymphocyte transformation is a term used for the first time by Nowell in 1960 and subsequently by Hirschorn to describe the morphological alterations that occur when small resting lymphocytes are transformed into lymphoblasts by exposure to the mitogen phytohaemagglutinin (PHA). Blastogenesis refers to the process of formation of large pyroninophile cells similar to blasts in lymphocyte cultures stimulated both with aspecific mitogens and with antigens. Lymphocyte activation is an in vitro technique commonly used to evaluate cellular immunity in subjects who are immunologically deficient, suffering from infectious and auto-immune diseases, or tumours. After incubation with mitogens, a number of complex biochemical events occur. The mitogens are substances that stimulate a large quantity of lymphocytes and do not require prior sensitization, which is necessary, on the contrary, in the case of antigen stimulation. The biochemical events comprise early phenomena correlated to the membrane, such as an increase in the synthesis of phospholipids, an increased permeability to bivalent cations, activation of adenincyclase and a simultaneous increase in intra-cell cAMP. Shortly after this, protein, RNA and finally DNA synthesis take place. It is the latter phenomenon, DNA synthesis, that precedes cell splitting, and is at the root of the majority of clinically important tests on lymphocyte activation. Convenience and habit have caused clinical immunologists to use DNA synthesis, rather than the earlier events such as increase in cAMP or phospholipid metabolism, as a marker of lymphocyte activation.

Lymphocyte transformation was originally evaluated by means of morphological testing of the percentage of lymphoblasts present in the culture. However, this method was abandoned due to the extreme variability and subjectivity of results. More accurate evaluation of DNA synthesis is now performed by pulse marking of the cultures using tritiated thymidine (3H-TdR), a radioactive nucleotidic precursor which is incorporated in the DNA during synthesis. The amount of tritiated thymidine taken up is determined by liquid phase scintillation (gamma counter). This method is at present generally accepted by the majority of clinical immunology laboratories. The scintillation count supplies data in counts per minute (cpm) or corrected for quenching into disintegrations per minute (dpm), which are used as a measurement of lymphocyte response. To express the data, the cpm of the stimulated cultures are divided by the cpm of the control cultures, and the result obtained gives a stimulation index.

Obviously, there are a number of variables that can affect the results of such a sensitive test system. These include the number of cells in the culture and their concentration, the geometry of the culture wells, contamination of the cultures with non-lymphoid cells or microorganisms, the dose of mitogen, the incubation time and the methods used to separate the lymphocytes and to harvest the cells. On a practical and clinical level the duration of the cultures and the concentration of the mitogen are extremely important, given that clinically evident deficiencies in cellular immunity are rarely absolute, and therefore the quantitative ratios for lymphocyte activation are of great importance. This is especially true when comparing the response of normal control subjects with that of a group of patients with alterations in their lymphocyte function. Using microcultures on plates with 96 wells and semi-automatic harvesting systems, it is possible to determine the dose-response and time-response kinetics for cultures stimulated both with mitogens and with antigens. Altered lymphocyte functionality may determine movements to the left or to the right of the time-response and dose-response curves. These shifts determine the optimum dose and the optimum time for lymphocyte response. Without such detailed analysis it is sometimes impossible to detect partial or slight defects in lymphocyte response. Cultures studied for a single period of time or using a single stimulating dose are often extremely imprecise. Confusion can also occur due to a lack of standardization in the presentation of data. The stimulation index is a ratio, and therefore considerable variations can result from alterations to the control cultures without mitogen (cpm expressed as the denominator). It would thus be preferable to give the results both as cpm of the stimulated cultures and as a stimulation index.

In recent years there has been progressive miniaturization of the cultures set up to evaluate lymphocyte proliferation. From 1 ml cultures in test tubes things have passed to 200 µl cultures on plates following the appearance of special automatic cell collection procedures (harvesters). Today it is possible to cultivate lymphocytes in suspended 20 µl hanging drops and to stimulate them with mitogens, antigens and allogenic leucocytes. Proliferation can be measured by incorporation of tritiated thymidine using a container in which the radio marked cultures are simply placed on a disk of filter paper, which is then washed and processed for the scintillation count (blot). This method has the dual advantage of using ten times less cells (10 - 40,000) and of simplifying the harvesting technique (British Technology Group of London system). The suspended 20 µl hanging drop technique is a practical solution to the necessity of performing multi-factor experiments, which are indispensable in order to identify the nature of each change seen in lymphocyte proliferation. 20 µl cultures in hanging drops can easily be obtained using human and animal lymphocytes in Terasaki plates. Compared with larger cultures, this method requires a great deal of attention in maintaining an adequate pH and perfect humidity inside the incubator during the culture period. Naturally, harvesting of the cells is extremely simple. To harvest a plate with 60 20 µl cultures, perform the blot, wash and process takes less than 2 minutes. This method therefore allows an enormous number of cultures for performing multi-factor experiments. This ability to perform multi-factor experiments (concentration, time, number of cells, autologous or heterologous serum, addition of dendritic cells, number of allogenic lymphocytes and so on) has made the use of special mathematical techniques necessary for data analysis.

These conventional methods have not shown themselves to be completely satisfactory. Their practical application has come up against a series of drawbacks. In effect, in spite of the fact that miniaturization of cultures, sophistication of harvesting methods, availability of dedicated software make it possible to obtain a better understanding of lymphocyte response to stimulus, conventional methods, due to their complexity and delicacy, are absolutely unsuitable for clinical use and are reserved for research laboratories alone.

It has now unexpectedly been found that adoption of the method for performing a stimulated lymphocyte transformation test, by flow cytometry, according to the present invention, makes it possible to overcome all the drawbacks indicated above, while at the same time giving further advantages that will be shown more clearly in the following.

The method for performing a stimulated lymphocyte transformation test according to the present invention, comprising taking a sterile heparinated sample; implanting the whole blood or separated lymphocytes in culture medium; incubation in a sterile incubator; setting up two cultures, one of which must contain the stimulant at concentrations such as to generate cell proliferation,
is characterized by the fact that incubation takes place at a temperature of between 25 and 45°C for a time of between 24 and 192 hours, and by the fact that it comprises the combination of the following operations:
- centrifugation at the end of the incubation;
- re-suspension in a lysing solution for erythrocytes;
- DNA staining;
- washing the leucocyte suspension with isotonic aqueous solution;
- centrifugation of the resulting leucocyte suspension;
- re-suspension of the cells in a solution lysing for leucocytes, in the presence of a DNA-specific fluorochrome, the lysing solution also optionally comprising reagents for optimization of lysis; and
- analysis of the samples, using flow cytometry.

The incubation at temperatures of between 25 and 45°C for a time of between 24 and 192 hours can be performed in a corked condition. Alternatively, it can be performed un-corked in culture mediums containing reagents that allow gaseous exchange, placing the culture in a sterile incubator with controlled humidity and carbon dioxide levels.

The cells can be re-suspended in leucocyte lysing solution, in the presence of a DNA-specific fluorochrome, the lysing solution also optionally containing reagents for optimization of lysis.

Alternatively, the cells can be re-suspended in fixing solution and subsequently washed and re-suspended in solution containing the DNA fluorochrome. As another alternative, the cells can be incubated with mono- or polyclonal antibodies -directly or indirectly conjugated with a fluorochrome - that are specific for the cell population under examination, and subsequently fixed and stained using DNA-specific fluorochrome.

The culture medium can be RPMI 1640 with 10% heat-inactivated fetal calf serum and 1% glutamine, said medium being used in an amount of 3 ml per culture. This culture medium can be freshly made or lyophilized.

The stimulant substances can be chosen, for example, from the group comprising phytohaemagglutinin (PHA), concavalline A (ConA) and pokeweed mitogen (PWM).

The erythrocyte lysing solution can be made up of bidistilled water containing NH₄Cl 8.28 g/l, KHCO₃ 1 g/l, mono- or tetrasodic EDTA 0.037 g/l.

The leucocyte lysing and DNA staining solution can consist of bidistilled water containing sodium citrate 0.1%, Nonidet NP-40 detergent 0.1%, and propidium iodide 50 µg/ml. The propidium iodide may also be substituted with ethydium bromide, DAPI and substances with similar properties.

The fixing solution can be made up of ethyl (or methyl) alcohol 50% (v/v) and isotonic phosphate buffer solution (PBS) 50% (v/v) and the DNA staining solution can be made up of propidium iodide 50 µg/ml and ribonuclease A 1mg/ml in isotonic phosphate buffer solution (PBS).

Attention is directed to the fact that the method of the present invention is applied to a fluid of human body after it has been removed from the body; the fluid is not returned to the same body. Moreover, the method of the invention is intended for obtaining information which merely provides intermediate results which on their own do not enable a decision to be made on the treatment necessary.

Compared with traditional methods using tritiated thymidine, the method according to the present invention shows the following additional advantages:
- much lower costs for reagents;
- better quality of final information (the present method gives results directly connected to the biological phenomenon);
- improved safety both for the environment and the place of work, as the use of radioactive materials is excluded;
- lower risk of culture contamination (the culture containers do not have to be opened during incubation);
- extreme simplicity and reproducibility of the method;
- results are substantially independent from the number of cells;
- whole blood cultures;
- very small quantity of blood is required.

So far a description of a general nature has been given of the present invention. With the aid of the following example, which refers to a specific embodiment thereof, further indications will now be given aimed at providing a clearer understanding of its objects, characteristics, advantages and methods of application.

### EXAMPLE

A commercial Kit for performing a clinical test for stimulated lymphocyte transformation using mitogens and flow cytometry according to the present invention, is made up as follows:
1) sterile container with rubber cap, containing RPMI 1640 culture medium at 10% heat-inactivated fetal calf serum and 1% glutamine. The medium can be freshly made or lyophilized. The volume of the medium, reconstituted if lyophilized, is of 3 ml for each culture. The rubber cap can be perforated with a needle. For each test two culture containers are provided, one of which also contains PHA (phytohaemagglutinin) in a concentration such as to generate lymphocyte proliferation. It is also possible to add, optionally in another commercial package, two more cultures containing optimum concentrations of ConA (concavalline A) and PWM (pokeweed mitogen) for more complete evaluation of lymphocyte function;
2) container containing powders or concentrated solution for obtaining an erythrocyte lysing solution for an amount of 5 ml per culture. The solution used is made up of bidistilled water containing NH₄Cl 8.28 g/l, KHCO₃ 1 g/l, mono- or tetrasodic EDTA 0.037 g/l;
3) container containing powders or concentrated solution, (or two containers, one containing powders and the other concentrated solution, to be mixed together) for obtaining a hypotonic leucocyte lysing solution containing DNA-specific fluorochrome. The solution used, in an amount of 2 ml per culture, is made up of bidistilled water containing sodium citrate 0.1%, "Nonidet NP-40" detergent 0.1%, propidium iodide 50 µg/ml. (The use of other fluorochromes is possible and provided for, according to the light source of the cytometer).

## Claims

1. A method for performing a stimulated lymphocyte transformation test, comprising taking a sterile heparinated sample, implanting the whole blood or separated lymphocytes in culture medium, incubation in a sterile incubator, setting up two cultures, one of which must contain the stimulant substance at concentrations such as to generate cell proliferation,
characterized by the fact that incubation takes place at a temperature of between 25 and 45°C for a time of between 24 and 192 hours, and by the fact that it comprises the combination of the following operations:
- centrifugation at the end of the incubation;
- re-suspension in a lysing solution for erythrocytes
- DNA staining;
- washing the leucocyte suspension with isotonic aqueous solution;
- centrifugation of the resulting leucocyte suspension;
- re-suspension of the cells in a solution lysing for leucocytes, in the presence of a DNA-specific fluorochrome;
- analysis of the samples, using flow cytometer.

2. The method according to claim 1, in which the incubation at temperatures of between 25 and 45°C for a time of between 24 and 192 hours is performed in a corked condition.

3. The method according to claim 1, in which the incubation at temperatures of between 25 and 45°C for a time of between 24 and 192 hours is performed un-corked in culture mediums containing reagents that allow gaseous exchange, placing the culture in a sterile incubator with controlled humidity and carbon dioxide levels.

4. The method according to any one of claims 1 to 3, in which the cells are re-suspended in leucocyte lysing solution, in the presence of a DNA-specific fluorochrome and in the presence of reagents for optimization of lysis.

5. The method according to any one of claims 1 to 3, in which the cells are re-suspended in fixing solution and subsequently washed and re-suspended in solution containing a DNA fluorochrome.

6. The method according to any one of claims 1 to 3, in which the cells are incubated with mono- or polyclonal antibodies - directly or indirectly conjugated with a fluorochrome - that are specific for the cell population under examination, and subsequently fixed and stained using DNA-specific fluorochrome.

7. The method according to claims 1 to 6, in which the culture medium is RPMI 1640 with 10% heat-inactivated fetal calf serum and 1% glutamine.

8. The method according to claim 7, in which the culture medium is either freshly made or lyophilized.

9. The method according to any one of the preceding claims, in which the stimulant substances are chosen from the group comprising phytohaemagglutinin (PHA), concavalline A (ConA), pokeweed mitogen (PWM) and combinations thereof.

10. The method according to any one of the preceding claims, in which the erythrocyte lysing solution is made up of bidistilled water containing NH₄Cl 8.28 g/l, KHCO₃ 1 g/l, mono- or tetrasodic EDTA 0.037 g/l.

11. Method according to claim 4, in which the leucocyte lysing and DNA colouring solution is made up of bidistilled water containing sodium citrate 0.1%, "Nonidet NP-40" detergent 0.1%, and propidium iodide 50 µg/ml.

12. The method according to claim 5 or 6, in which the fixing solution is made up of ethyl alcohol 50% (v/v) and isotonic phosphate buffer solution (PBS) 50% (v/v), and the DNA staining solution is made up of propidium iodide 50 µg/ml and ribonuclease A 1 mg/ml in isotonic phosphate buffer solution (PBS).

13. A method for performing a stimulated lymphocyte transformation test, using flow cytometry, as described, exemplified and claimed above.
